# EUROPÄISCHE PATENTANMELDUNG

(11) **EP 0 631 761 A1**
(43) Veröffentlichungstag der Anmeldung: **04.01.1995**
(21) Anmeldenummer: 94101029.0
(22) Anmeldetag: 25.01.1994
(51) Int. Cl.: A61D 9/00

(54) **Verband für Gliedmassen**

(30) Priorität: 01.07.1993 DE 9309798 U
(71) Anmelder: R. DEMHARTNER GmbH & Co. KG, 84051 Essenbach (DE)
(72) Erfinder: Demhartner, Rudolf, D-84051 Essenbach (DE)
(74) Vertreter: Gustorf, Gerhard, Dipl.-Ing.

(57) **Zusammenfassung**

Gegenstand der Erfindung ist ein Verband (14) für Gliedmaßen, insbesondere ein Fesselverband für Huftiere. Er besteht aus einem im wesentlichen rechteckigen, mehrlagigen Umschlagpolster (20), an dessen Außenseite ein elastisches Band (22) angebracht ist. Der Verband (14) kann somit rasch angelegt werden und ist gegen Verrutschen weitgehend gesichert.

## Beschreibung

Die Erfindung betrifft einen Verband für Gliedmaßen, insbesondere einen Fesselverband für Huftiere.

Huftiere und insbesondere Pferde benötigen gelegentlich einen Fesselverband, um eine Wunde abzudecken. Hierfür wird die Fessel im Bereich der Verletzung mit einer Stoffbinde umwickelt, deren freies Ende dann mit Hilfe eines Klebestreifens oder eines elastischen Klemmstücks befestigt wird. Nachteilig ist dabei, daß ein derartiger Fesselverband nur verhältnismäßig umständlich angelegt werden kann und häufig nach kurzer Zeit verrutscht, insbesondere bei heftigen Bewegungen.

Der Erfindung liegt die Aufgabe zugrunde, einen Verband für Gliedmaßen zur Verfügung zu stellen, der rasch angelegt werden kann und gegen ein Verrutschen weitgehend gesichert ist.

Erfindungsgemäß wird diese Aufgabe gelöst durch ein im wesentlichen rechteckiges, mehrlagiges Umschlagpolster mit wenigstens einem in Umschlagrichtung verlaufenden, elastischen Band.

Das am Umschlagpolster befestigte Band, beispielsweise ein herkömmliches, aufgenähtes oder eingeschweißtes Gummiband, hat den erheblichen Vorteil, daß der angelegte Verband im Bereich des elastischen Bandes fest gegen den zu schützenden Körperteil gedrückt wird, so daß er auch bei heftigen Bewegungen seinen Platz beibehält und die Wunde schützt. In aller Regel werden zwei zueinander parallele elastische Bänder vorgesehen, die entsprechend der Anatomie des Körperteils angeordnet sind und so eine Anpassung des Verbandes an diesen gewährleisten. Der Verband wird so angelegt, daß sich die zu behandelnde Stelle, z. B. die Verletzung, im Bereich zwischen den beiden elastischen Bändern befindet, durch die er sicher gehalten wird.

Es ist günstig, wenn an einem rechtwinklig zur Umschlagrichtung verlaufenden Rand des Umschlagpolsters wiederverschließbare Klebestreifen angebracht sind. Auf diese Weise steht ein Schnellverband zur Verfügung, der rasch angelegt und bei Bedarf geöffnet und mit denselben Klebestreifen auch wieder geschlossen werden kann.

In Weiterbildung der Erfindung hat das mehrlagige Umschlagpolster an der Innenseite eine Faservliesfolie, die eine Füllung aus Zellstoffwatte abdeckt.

Das Umschlagpolster kann an seiner Außenseite durch eine undurchlässige Kunststoff-Folie abgedeckt sein, die beispielsweise aus Polyethylen besteht. Die Wunde wird auf diese Weise gegen das Eindringen von Feuchtigkeit und Schmutzpartikeln geschützt. Zudem wird ein wärmender Effekt erzielt.

Wenn der Verband zum Kühlen dienen soll, beispielsweise zur Verhinderung eines Wärmestaus an den Pferdefesseln nach einem Rennen, ist es günstig, wenn das Umschlagpolster an der Außenseite durch eine atmungsaktive Folie abgedeckt ist, beispielsweise eine sogenannte Trichterfolie. Diese gestattet dann ein Ableiten der Wärme nach außen.

Die Erfindung ist nachstehend an zwei Ausführungsbeispielen erläutert, die in der Zeichnung dargestellt sind. Es zeigen:
Figur 1 die Ansicht eines Pferdefußes beim Anlegen eines Schnellverbandes gemäß der Erfindung,
Figur 2 die Ansicht eines Schnellverbandes von außen und
Figur 3 eine Schnittdarstellung in der Ebene III-III der Figur 2.

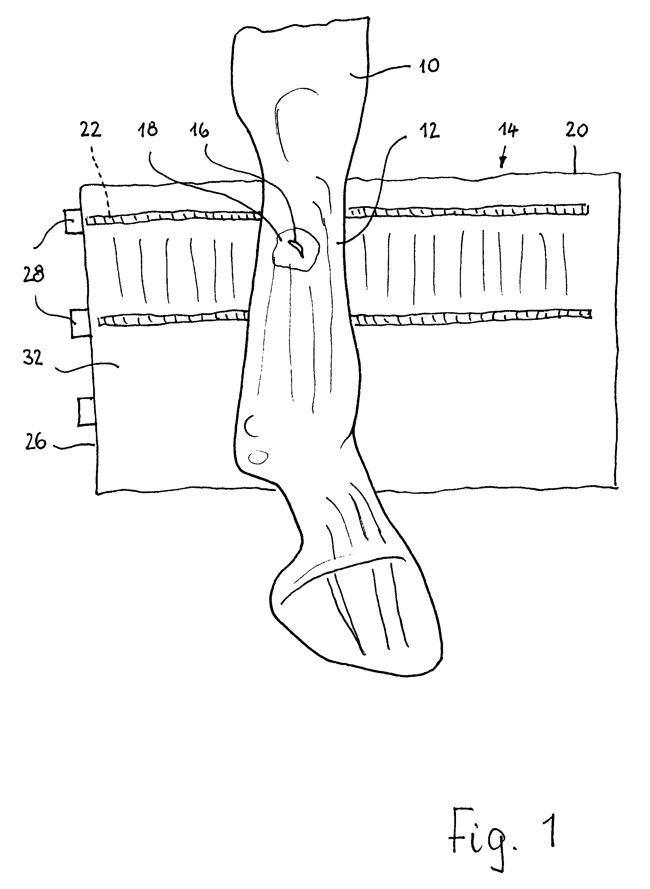

Figur 1 zeigt einen Pferdefuß 10, der im Bereich der Fessel 12 durch einen Verband 14 gemäß der Erfindung abgedeckt werden soll, beispielsweise zur Heilung einer Wunde 16. Vor dem Anlegen des Verbandes 14 kann die Wunde 16 mit einer Salbe 18 bestrichen werden; es ist jedoch auch möglich, den Verband 14 an der entsprechenden Stelle seiner Innenseite mit der Salbe zu bestreichen oder mit einer Heillösung zu tränken.

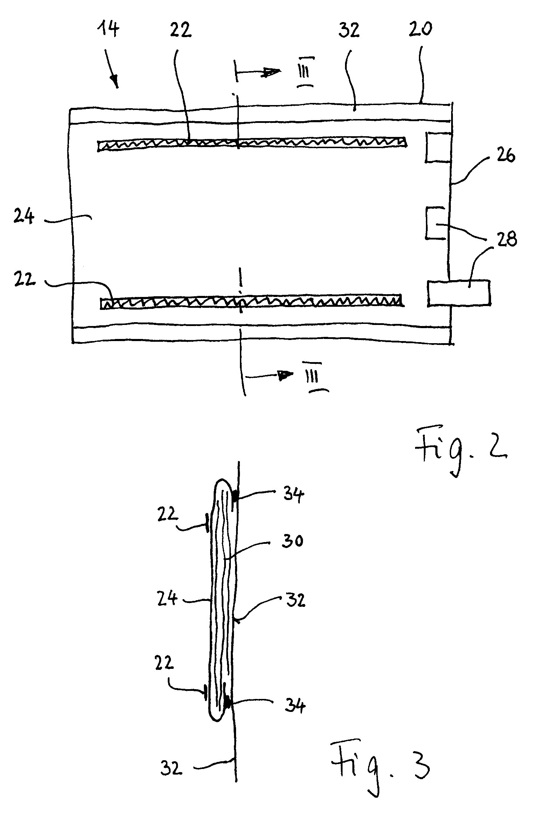

Der Verband 14 besteht aus einem rechteckigen, mehrlagigen Umschlagpolster 20, auf dessen Außenseite zwei in Umschlagrichtung verlaufende, zueinander parallele und elastische Bänder 22 aufgenäht sind (vgl. Figuren 2 und 3). Wie bei Windeln an sich bekannt, wird die Außenseite des Umschlagpolsters 20 durch eine Folie 24 gebildet, die als eine undurchlässige Kunststoff-Folie, z. B. aus Polyethylen, oder als atmungsaktive Folie, beispielsweise Trichterfolie, ausgebildet sein kann. An einem rechtwinklig zur Umschlagrichtung verlaufenden Rand 26 des Umschlagpolsters 20 sind an der Folie 24 wiederverschließbare Klebestreifen 28 angebracht.

Das mehrlagige Umschlagpolster 20 hat eine Füllung 30 aus ungebleichter Zellstoffwatte, die in etwa 10 bis 20 Lagen eingesetzt ist. Die Füllung 30 wird zur Innenseite hin durch eine thermogebundene Faservliesfolie 32 abgedeckt. In Figur 3 ist angedeutet, daß die äußere Folie 24 mit ihren beiden in Wickelrichtung verlaufenden Längsrändern nach innen auf die Füllung 30 umgelegt ist; die Längsränder sind dann über Leimrauten 34 mit der Faservliesfolie 32 verklebt.

Das elastische Band 22 ist so auf die äußere Folie 24 aufgenäht, daß sich das Umschlagpolster 20 im Bereich des elastischen Bandes 22 zusammenzieht. Beim Anlegen des Verbandes 14 drücken dann die elastischen Bänder 22 das Umschlagpolster 20 gegen die zu schützende Fessel 12, wodurch ein sicherer Halt gewährleistet wird. Nach dem Umlegen des Verbandes 14 wird dieser mittels der wiederverschließbaren Klebestreifen 28 verschlossen.

Der gemäß der Erfindung ausgebildete Schnellverband kann in unterschiedlichen Abmessungen bereitgehalten werden, wobei auch die elastischen Bänder 22 in unterschiedlicher Zahl und mit unterschiedlichem Abstand voneinander angebracht sein können, abhängig von dem vorgesehenen Verwendungszweck entsprechend der Anatomie der zu schützenden Gliedmaßen. Selbstverständlich eignet sich dieser Schnellverband nicht nur für Pferde, sondern auch zum Schützen von Gliedmaßen anderer Lebewesen.

## Patentansprüche

1. Verband für Gliedmaßen, insbesondere Fesselverband für Huftiere, gekennzeichnet durch ein im wesentlichen rechteckiges, mehrlagiges Umschlagpolster (20) mit wenigstens einem in Umschlagrichtung verlaufenden, elastischen Band (22).

2. Verband nach Anspruch 1, dadurch gekennzeichnet, daß an einem rechtwinklig zur Umschlagrichtung verlaufenden Rand (26) des Umschlagpolsters (20) wiederverschließbare Klebestreifen (28) angebracht sind.

3. Verband nach Anspruch 1 oder 2, dadurch gekennzeichnet, daß das mehrlagige Umschlagpolster (20) an der Innenseite eine Faservliesfolie (32) hat, die eine Füllung (30) aus Zellstoffwatte abdeckt.

4. Verband nach Anspruch 3, dadurch gekennzeichnet, daß das Umschlagpolster (20) an der Außenseite durch eine undurchlässige Kunststoff-Folie (24) abgedeckt ist.

5. Verband nach Anspruch 3, dadurch gekennzeichnet, daß das Umschlagpolster (20) an der Außenseite durch eine atmungsaktive Folie abgedeckt ist.

6. Verband nach einem der vorhergehenden Ansprüche, dadurch gekennzeichent, daß das elastische Band (22) auf die Außenseite des Umschlagpolsters (20) aufgenäht ist.
